# EUROPEAN PATENT APPLICATION

(11) **EP 4 844 053 A1**
(43) Date of publication of application: **25.11.2026**
(21) Application number: 24926957.2
(22) Date of filing: 23.12.2024
(51) Int. Cl.: C12M 1/34

(54) **CELL OBSERVATION DEVICE, METHOD FOR OPERATING CELL OBSERVATION DEVICE, AND PROGRAM FOR OPERATING CELL OBSERVATION DEVICE**

(30) Priority: 26.02.2024 JP 2024026481
(71) Applicant: FUJIFILM Corporation, Tokyo 106-8620 (JP)
(72) Inventor: MATSUBARA, Kenta, Ashigarakami-gun, Kanagawa 258-8577 (JP)
(74) Representative: Hoffmann Eitle
(86) International application number: PCT/JP2024/045505
(87) International publication number: WO 2025/182272

(57) **Abstract**

A cell observation apparatus includes a processor configured to acquire flow channel information, the flow channel information being information relating to a flow channel through which a cell suspension including cells flows, and determine an image acquisition condition for acquiring an image of the cells in the flow channel, based on the flow channel information.

## Description

### BACKGROUND OF THE INVENTION

### 1. Field of the Invention

The technology of the present disclosure relates to a cell observation apparatus, an operation method for the cell observation apparatus, and an operation program for the cell observation apparatus.

### 2. Description of the Related Art

A cell culture system for culturing cells using a culture vessel is known (see, for example, WO2015/069616A). The culture vessel accommodates a cell suspension in which the cells are suspended in a culture medium. Cell culture requires appropriate determination of a process condition of the culturing process, such as temperature control of the culture vessel, concentration control of the cell suspension in the culture vessel, and control of components in the culture medium, such as vitamins and amino acids.

### SUMMARY OF THE INVENTION

To appropriately determine the process condition, it is necessary to ascertain cell quality, which is the state of cells being cultured, and take the ascertained cell quality into consideration. To this end, cells being cultured are extracted from the culture vessel, and the extracted cells are transferred to a vessel such as a petri dish and observed to ascertain the cell quality. However, such an offline inspection is labor-intensive and time-consuming. Therefore, an inline inspection technique is being studied in which cells being cultured are observed inline while flowing through a flow channel. As a cell observation apparatus, a phase-contrast microscope, three-dimensional image measurement using digital holography technology, and the like are used.

However, there is a concern that in-line observation of cells may degrade image quality because a flow channel is captured in an image in addition to the cells to be observed. If the image quality is degraded, cell quality cannot be ascertained with high accuracy, which may hinder appropriate determination of process control conditions.

The technology of the present disclosure provides a cell observation apparatus, an operation method for the cell observation apparatus, and an operation program for the cell observation apparatus, each capable of acquiring a cell image having improved image quality compared to the related art.

To achieve the above object, a cell observation apparatus according to the technology of the present disclosure includes a processor configured to acquire flow channel information, the flow channel information being information relating to a flow channel through which a cell suspension including cells flows, and determine an image acquisition condition for acquiring an image of the cells in the flow channel, based on the flow channel information.

Preferably, the image acquisition condition includes at least one of an imaging condition for capturing the image of the cells in the flow channel or an image processing condition for performing image processing.

Preferably, the imaging condition includes at least one of a wavelength of illumination light for illuminating the flow channel or an exposure condition.

Preferably, the flow channel information includes at least one of information relating to a structure of the flow channel or information relating to a characteristic of the flow channel.

Preferably, the characteristic of the flow channel includes a refractive index of a flow channel member constituting the flow channel.

Preferably, the cell observation apparatus includes an imaging device configured to capture the image of the cells, and the imaging device is an image measurement device using digital holography technology.

Preferably, the cell observation apparatus includes an imaging device configured to capture the image of the cells, and a detection mechanism configured to detect passage of the cells in a section on an upstream side of the imaging device in the flow channel and detect at least one of a passage timing of the cells that pass, a number of cells that pass, a density of the cells that pass, or a size of a cell aggregate that passes, and the processor is configured to determine the image acquisition condition based on a detection result of the detection mechanism.

Preferably, the processor is configured to determine the image acquisition condition based on, in addition to the flow channel information, information on at least one of a type of the cells or a type of a culture medium used for the cell suspension.

Preferably, the processor further has a function of determining a process condition of a culturing process for the cells, based on the image.

Preferably, the flow channel is a flow channel through which the cell suspension that is extracted from a culture vessel for culturing the cells and that includes a cell being cultured flows.

An operation method for a cell observation apparatus according to the technology of the present disclosure is an operation method for a cell observation apparatus including a processor, the operation method including, by the processor: acquiring flow channel information, the flow channel information being information relating to a flow channel through which a cell suspension including cells flows; and determining an image acquisition condition for acquiring an image of the cells in the flow channel, based on the flow channel information.

An operation program for a cell observation apparatus according to the technology of the present disclosure is an operation program for a cell observation apparatus including a processor, the operation program causing the processor to execute a process including: acquiring flow channel information, the flow channel information being information relating to a flow channel through which a cell suspension including cells flows; and determining an image acquisition condition for acquiring an image of the cells in the flow channel, based on the flow channel information.

The technology of the present disclosure enables acquisition of a cell image having improved image quality compared to the related art.

### BRIEF DESCRIPTION OF THE DRAWINGS

Fig. 1 is a diagram illustrating an overview of a cell culture system with a cell observation apparatus incorporated therein;
Fig. 2 is a block diagram illustrating a hardware configuration of the cell observation apparatus;
Fig. 3 is a diagram illustrating an imaging unit using digital holography technology;
Fig. 4 is a diagram conceptually illustrating a principle of generation of interference fringes and an interference fringe image;
Fig. 5 is a diagram illustrating a positional relationship between a flow channel member and the imaging unit;
Fig. 6 is an explanatory diagram of an image acquisition condition;
Fig. 7 is a diagram illustrating a method of determining the amount of illumination light according to the transmittance of the flow channel member;
Fig. 8 is a diagram illustrating a method of determining the amount of illumination light according to the dimensions of a flow channel;
Fig. 9 is a diagram illustrating an image processing condition determined based on flow channel information;
Fig. 10 is a flowchart illustrating an operation procedure of the cell observation apparatus;
Fig. 11 is a diagram illustrating an overview of a cell observation apparatus according to a second embodiment;
Fig. 12 is a diagram illustrating cell-number-related information detected based on a photosensor;
Fig. 13 is a diagram illustrating an example of use of cell passage timings;
Fig. 14 is a diagram illustrating an example of a high cell density state;
Fig. 15 is a diagram illustrating a cell aggregate;
Fig. 16 is a diagram illustrating a method of determining the amount of illumination light according to a cell density or the like;
Fig. 17 is a diagram illustrating a method of determining a wavelength of illumination light according to the size of the cell aggregate; and
Fig. 18 is a diagram illustrating a cell observation apparatus having a function of determining a process control condition.

### DESCRIPTION OF THE PREFERRED EMBODIMENTS

### First Embodiment

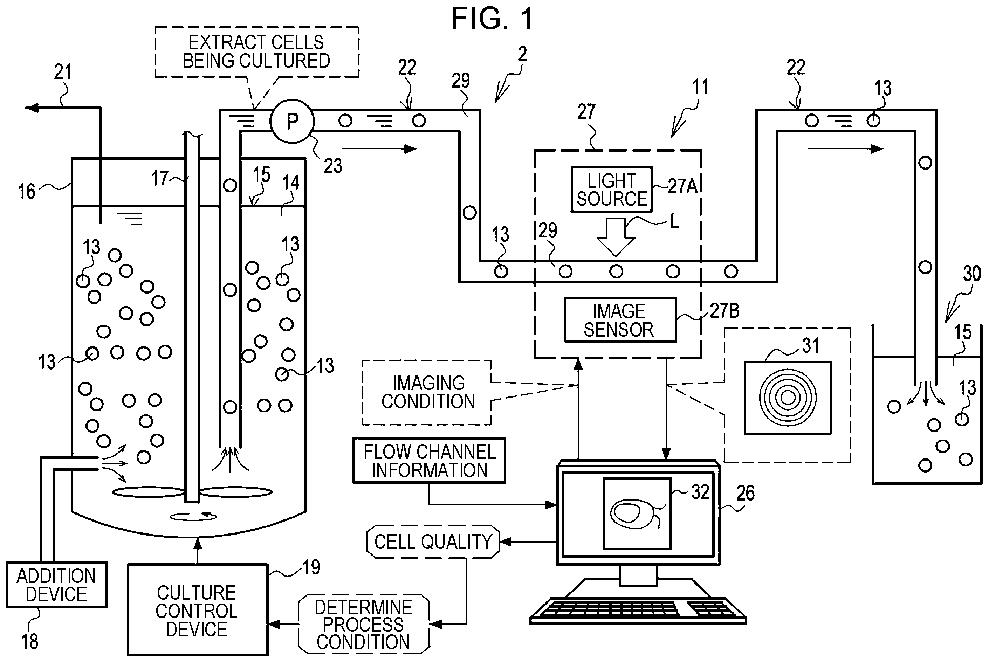

In Fig. 1, a cell observation apparatus 11 is incorporated in a cell culture system 2. The cell culture system 2 includes a culture vessel 16 for culturing cells 13 therein. The culture vessel 16 accommodates a cell suspension 15 obtained by suspending the cells 13 in a culture medium 14. In the cell culture system 2, the culturing process for the cells 13 is appropriately managed.

To appropriately manage the culturing process, for example, a process condition, such as a culture condition, needs to be set to an appropriate condition. Examples of the culture condition include environmental conditions of the culture vessel 16, component replenishment conditions of the culture medium 14, and a replacement timing of the culture medium 14. The environmental conditions of the culture vessel 16 are the temperature of the culture vessel 16, the stirring speed of the cell suspension 15 by a stirring device 17, and the like. The component replenishment conditions of the culture medium 14 are an amount, a timing, and the like of replenishment of the components included in the culture medium 14 by an addition device 18. The components of the culture medium 14 include a carbon source, vitamins, amino acids, and the like. As management of the culturing process, examples of state management of the cell suspension 15 include removal of debris (such as dead cells and residues) in the cell suspension 15 and replacement of the culture medium 14. A culture control device 19 executes such control of the culturing process.

Cultured cells 13 or a product produced by the cells 13 is recovered from the culture vessel 16 through a culture product recovery path 21 from the culture vessel 16 toward a subsequent step. Examples of the process condition also include a recovery timing of the cultured cells 13.

In addition to the culture product recovery path 21, an observation path 22 is provided for the culture vessel 16. The observation path 22 is provided with a pump 23, and the pump 23 extracts the cell suspension 15 including the cells 13 being cultured from the culture vessel 16. The cells 13 being cultured, which flow through a flow channel 29 of the observation path 22, are observed by the cell observation apparatus 11.

The cell observation apparatus 11 is used to ascertain the cell quality of the cells 13 being cultured in order to appropriately manage the culturing process. That is, the cell observation apparatus 11 performs inline inspection of the cells 13 flowing through the flow channel 29 of the observation path 22. Then, the necessity to change the process condition including the culture condition and the content of the change are determined based on the cell quality of the cells 13 being cultured. Furthermore, based on the cell quality, operations such as determining a timing for removing debris, a timing for replacing the culture medium 14, and a timing for recovering a culture product are performed.

The cell observation apparatus 11 includes a control device 26 and an imaging unit 27. The imaging unit 27 is disposed in the observation path 22. The cells 13 that flow through the observation path 22 and are imaged are stored in a storage portion 30. For example, some of the cells 13 in the storage portion 30 are returned to the culture vessel 16 and others are discarded according to the cell state.

The imaging unit 27 has a light source 27A that emits illumination light L for illuminating the cells 13 to be observed, and an image sensor 27B that captures an optical image of the illuminated light source 27A. As an example, the cell observation apparatus 11 is an image measurement device using digital holography technology. The cell observation apparatus 11 measures, for example, an image representing the three-dimensional shape of the cells 13. The image representing the three-dimensional shape of the cells 13 is also referred to as a three-dimensional image. Observing the three-dimensional shape of the cells 13 enables more accurate ascertainment of the cell quality. The imaging unit 27 is an example of an "imaging device" according to the technology of the present disclosure.

The image sensor 27B captures, as an optical image, an interference fringe image 31 representing an interference state between object light and reference light. The control device 26 controls driving of the imaging unit 27 and executes image processing for reconstructing a cell image 32 representing the three-dimensional shape of the cells 13, based on the interference fringe image 31. The control device 26 determines an image acquisition condition for acquiring the cell image 32. The image acquisition condition includes an imaging condition of the imaging unit 27 and an image processing condition for reconstructing the interference fringe image 31 into the cell image 32.

In a section of the observation path 22 where the imaging unit 27 is disposed, the cell observation apparatus 11 acquires flow channel information, which is information relating to the flow channel 29 through which the cell suspension 15 flows. Then, the cell observation apparatus 11 determines the image acquisition condition based on the acquired flow channel information.

The cell quality of the cells 13 being cultured is ascertained based on the cell image 32 acquired by the cell observation apparatus 11, and the process condition including the culture condition of the culturing process described above is determined based on the ascertained cell quality. The culturing process is controlled by the culture control device 19 in accordance with the determined process condition.

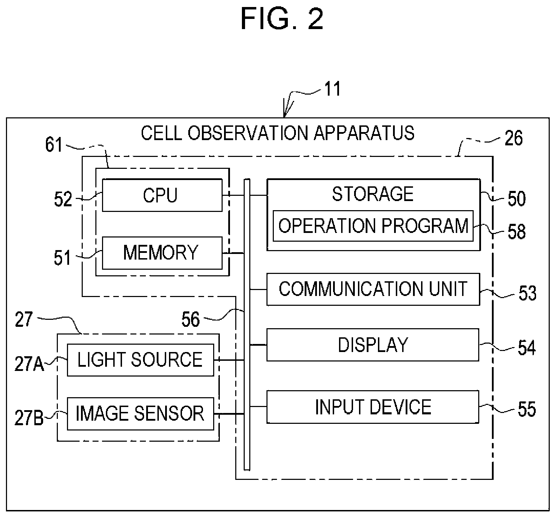

As illustrated in Fig. 2, in the cell observation apparatus 11, the control device 26 is constituted by, for example, a computer. The control device 26 includes a storage 50, a memory 51, a central processing unit (CPU) 52, a communication unit 53, a display 54, and an input device 55. These are interconnected via a busline 56.

The storage 50 is a hard disk drive installed in a computer constituting the control device 26 or connected via a cable or a network. Alternatively, the storage 50 is a disk array in which a plurality of hard disk drives are arranged. The storage 50 stores a control program such as an operating system, various application programs, various data associated with these programs, and the like. A solid-state drive may be used instead of the hard disk drive.

The memory 51 is a work memory for the CPU 52 to execute processing. The CPU 52 loads a program stored in the storage 50 into the memory 51 and executes processing according to the program, thereby integrally controlling each part of the computer.

The storage 50 further stores an operation program 58 according to the technology of the present disclosure. The operation program 58 is an application program for causing the computer to function as the cell observation apparatus 11. The CPU 52 executes the operation program 58, thereby functioning as a processor 61 according to the technology of the present disclosure in cooperation with the memory 51.

The communication unit 53 is a network interface that performs transmission control of various kinds of information via a network such as a local area network (LAN). The display 54 displays various screens. The computer constituting the control device 26 receives input of operation instructions from the input device 55 through the various screens. The input device 55 is a keyboard, a mouse, a touch panel, or the like.

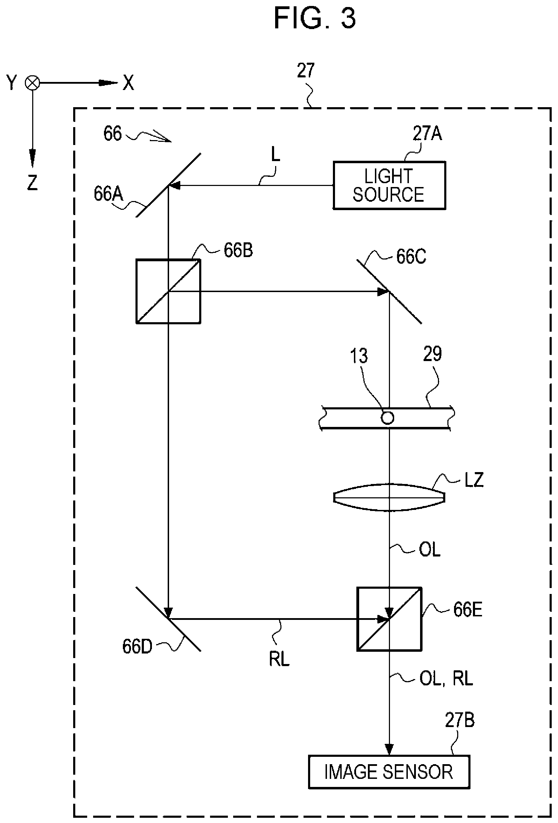

As illustrated in Fig. 3, the imaging unit 27 includes an imaging optical system 66 in addition to the light source 27A and the image sensor 27B. As is well known, digital holography is a technique for recording phase information of light in addition to intensity information of light of an object to be observed by using coherence of light, and for reproducing a three-dimensional image of the object by using the recorded intensity information and phase information.

In digital holography, coherent laser light is used as the illumination light L in order to utilize interference of light. The light source 27A is a laser light source that emits laser light as the illumination light L. The image sensor 27B is a two-dimensional image sensor having an image pick-up surface provided with a two-dimensional array of light-receiving elements. For example, a complementary metal-oxide semiconductor (CMOS) image sensor or the like is used.

The imaging optical system 66 is disposed in an optical path extending from the light source 27A to the image sensor 27B. As an example, the imaging optical system 66 includes a first reflection mirror 66A, a beam splitter 66B, a second reflection mirror 66C, an imaging lens LZ, a third reflection mirror 66D, and a beam combiner 66E. The first reflection mirror 66A reflects the illumination light L emitted from the light source 27A toward the beam splitter 66B. The beam splitter 66B separates the illumination light L into an object light path directed toward the second reflection mirror 66C and a reference light path directed toward the third reflection mirror 66D.

In the object light path including the second reflection mirror 66C, the flow channel 29 through which the cells 13 pass is disposed, and the cells 13 are irradiated with the illumination light L reflected by the second reflection mirror 66C. The illumination light L that has passed through the flow channel 29 and the cells 13 is incident on the imaging lens LZ as object light OL. The imaging lens LZ condenses the object light OL and forms an optical image of the condensed object light OL on the image pick-up surface of the image sensor 27B through the beam combiner 66E.

On the other hand, the illumination light L traveling through the reference light path from the beam splitter 66B toward the third reflection mirror 66D is incident on the third reflection mirror 66D without passing through the flow channel 29 through which the cells 13 flow. The illumination light L reflected by the third reflection mirror 66D is incident on the beam combiner 66E as reference light RL. Then, the reference light RL is incident on the image sensor 27B through the beam combiner 66E.

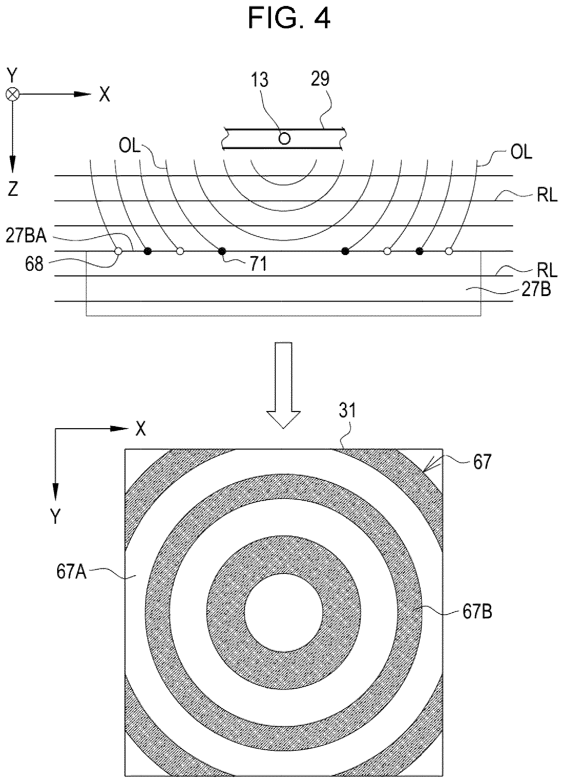

As conceptually illustrated in Fig. 4, the object light OL transmitted through the flow channel 29 and the cells 13 is diffracted by the cells 13 and substances constituting the flow channel 29, resulting in a curved wavefront. In contrast, the reference light RL not passing through the flow channel 29 and the cells 13 is not diffracted by the cells 13 and the like, resulting in a plane wave. The object light OL has a phase difference relative to the reference light RL, and the phase difference has a magnitude according to the refractive indices of the cells 13, the substances constituting the flow channel 29, and the like. The object light OL and the reference light RL interfere with each other on an image pick-up surface 27BA of the image sensor 27B, thereby generating interference fringes 67. The image sensor 27B captures an image of the interference fringes 67 and outputs the interference fringe image 31.

The pixel values of the respective pixels in the interference fringe image 31 indicate the intensities of the interference fringes 67. That is, the interference fringe image 31 is a two-dimensional distribution of the intensities of the interference fringes 67. The intensities of the interference fringes 67 vary in the image pick-up surface 27BA (X-Y plane) in accordance with the phase difference between the object light OL and the reference light RL.

Among lines representing the object light OL and the reference light RL, solid lines indicate wavefronts of the object light OL and the reference light RL at the maximum amplitude. In contrast, broken lines indicate wavefronts of the object light OL and the reference light RL at the minimum amplitude. White points 68 illustrated on the image pick-up surface 27BA are portions where wavefronts of the object light OL and the reference light RL are aligned to reinforce each other. The portions corresponding to the white points 68 appear as bright portions 67A in the interference fringes 67. In contrast, black points 71 illustrated on the image pick-up surface 27BA are portions where wavefronts of the object light OL and the reference light RL are shifted by a half wavelength to weaken each other. The portions corresponding to the black points 71 appear as dark portions 67B in the interference fringes 67.

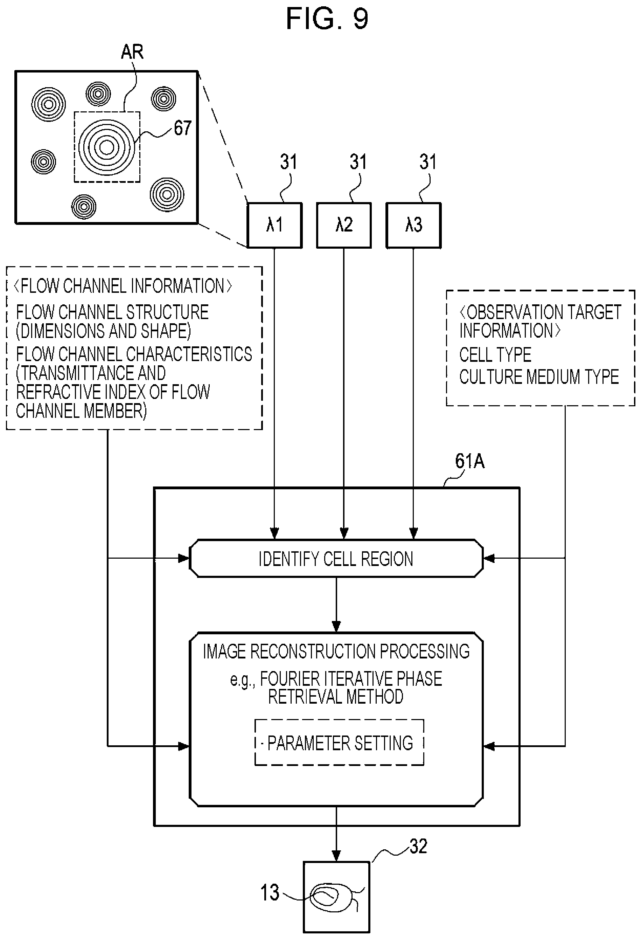

For example, depending on an image reconstruction method for digital holography, a plurality of interference fringe images 31 captured while varying an optical path difference between the object light OL and the reference light RL are used. Also regarding the illumination light L, interference fringe images 31 captured using, for example, three or more illumination lights L having different wavelengths (see interference fringe images of wavelengths λ1, λ2, and λ3 illustrated in Fig. 9) are acquired. Based on the plurality of interference fringe images 31, image reconstruction of the cell image 32 representing the three-dimensional shape of the cells 13 is performed.

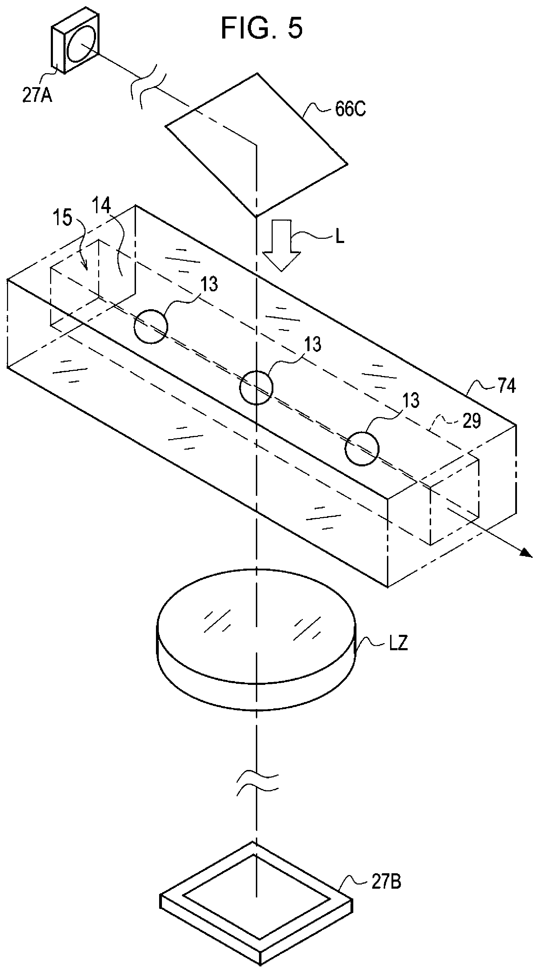

As illustrated in Fig. 5, the flow channel 29 is formed in, for example, a flow channel member 74. The flow channel member 74 is a plate-shaped member transparent to the illumination light L, and is made of, for example, plastic. The flow channel 29 is defined by, for example, a groove formed in part of the flow channel member 74, and the formed groove is sealed with a cover. The flow channel member 74 is also referred to as a microchannel device or the like.

The illumination light L is transmitted through the flow channel member 74 and the cells 13. Since the cell suspension 15 including the cells 13 flows through the flow channel 29, the illumination light L is also transmitted through the culture medium 14 other than the cells 13 in the cell suspension 15. In the example illustrated in Fig. 5, a cross-sectional shape of the flow channel 29 orthogonal to a flow direction of the flow channel 29 is rectangular. It is to be understood that the cross-sectional shape of the flow channel 29 may be circular. The flow channel member 74 may be a tube or the like instead of a plate-shaped member.

As an example, the imaging unit 27 repeatedly performs still-image capture of the interference fringe image 31 at preset intervals. Accordingly, interference fringe images 31 in which the cells 13 flowing through the flow channel 29 appear are captured. It is to be understood that the interference fringe images 31 may be recorded as a moving image.

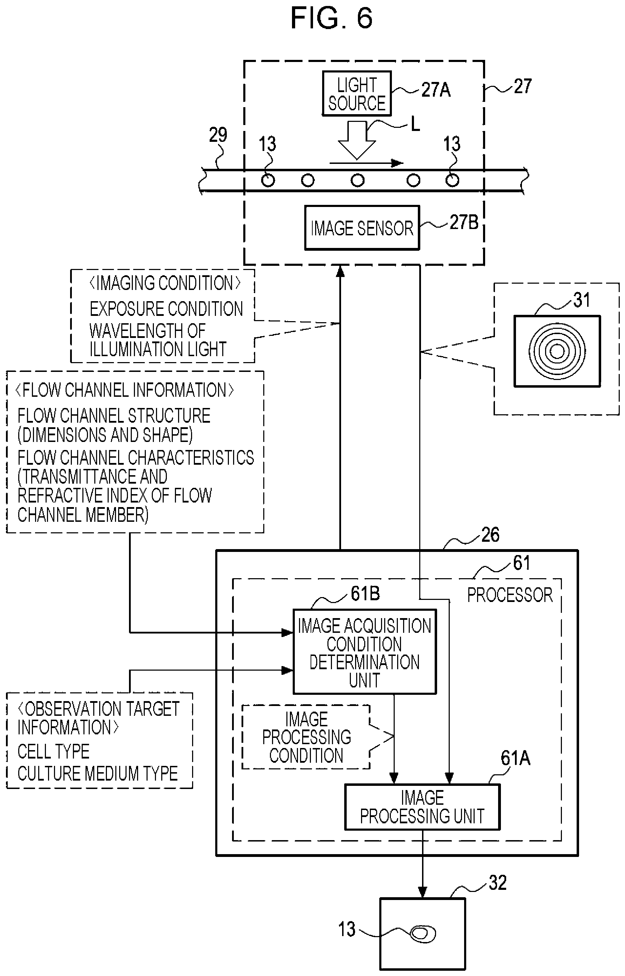

As illustrated in Fig. 6, the processor 61 of the control device 26 functions as an image processing unit 61A and an image acquisition condition determination unit 61B. The image processing unit 61A acquires an interference fringe image 31 from the imaging unit 27 and reconstructs a cell image 32 based on the acquired interference fringe image 31. The image acquisition condition determination unit 61B determines an image acquisition condition including an imaging condition of the imaging unit 27 and an image processing condition of the image processing unit 61A.

The image acquisition condition determination unit 61B determines an imaging condition and an image processing condition based on the flow channel information. The flow channel information includes information relating to the structure of the flow channel and information relating to the characteristics of the flow channel. The information relating to the structure of the flow channel includes the shape and dimensions of the flow channel 29. The information may include the shape and dimensions of the flow channel member 74. The information relating to the characteristics of the flow channel 29 includes the transmittance, the refractive index, and the like of the flow channel member 74. The transmittance and the refractive index are characteristics determined according to the material of the flow channel member 74.

Further, the image acquisition condition determination unit 61B acquires, as observation target information, the type of the cells 13 and the type of the culture medium 14. The image acquisition condition determination unit 61B determines the image acquisition condition based on the observation target information in addition to the flow channel information.

Examples of the imaging condition include an exposure condition and the wavelength of the illumination light L. Given the same amount of the illumination light L, the exposure amount of the image sensor 27B decreases as the transmittance of the flow channel member 74 decreases. Also given the same amount of the illumination light L, the exposure amount of the image sensor 27B decreases as the dimensions of the flow channel 29 increase. This is because, for example, if the cross-sectional area of the flow channel 29 determined by the width and the height of the flow channel 29 is large, the amount of diffusion and the amount of attenuation of the illumination light L increase accordingly.

If the exposure amount is too low, the contrast of the cell image 32 decreases. The exposure amount is determined according to an exposure condition including an exposure time, which is an accumulation time for accumulating incident light in the image sensor 27B, and the amount of the illumination light L of the light source 27A. The image acquisition condition determination unit 61B determines an exposure condition of the imaging unit 27, including the exposure time and the amount of illumination light of the light source 27A, based on the transmittance of the flow channel member 74 so as to obtain an appropriate exposure amount.

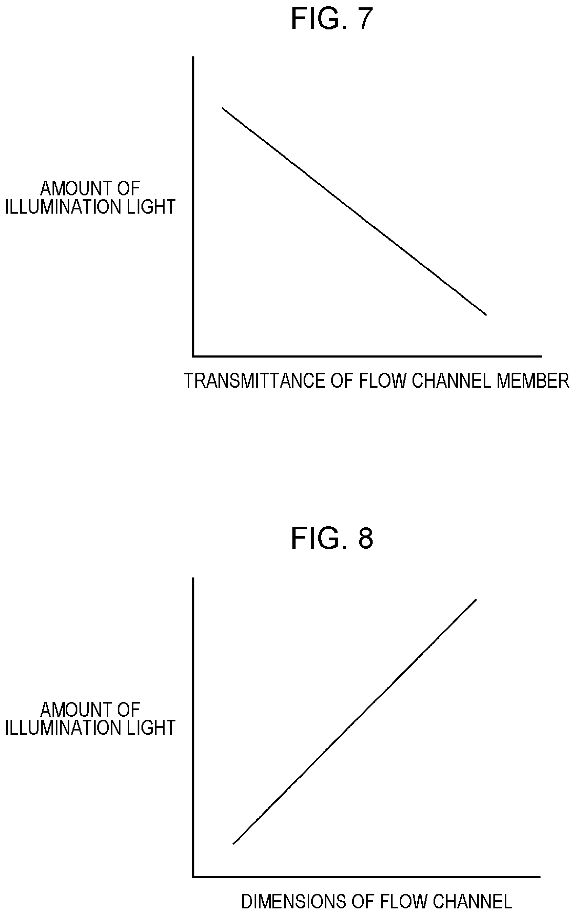

For example, as illustrated in Fig. 7, the image acquisition condition determination unit 61B increases the amount of the illumination light L as the transmittance of the flow channel member 74 decreases. As illustrated in Fig. 8, the image acquisition condition determination unit 61B increases the amount of the illumination light L as the dimensions of the flow channel 29 increase. Controlling such an exposure condition allows an appropriate exposure amount to be obtained regardless of the transmittance of the flow channel member 74, the dimensions of the flow channel 29, and the like. Accordingly, the decrease in the contrast of the cell image 32 is suppressed, resulting in an improvement in image quality. For example, the storage 50 stores table data or functions indicating the relationships illustrated in Figs. 7 and 8. While referring to such data, the image acquisition condition determination unit 61B executes control of an exposure condition based on the flow channel information.

Furthermore, if scattering of the illumination light L in an observation target is significant, the image quality is degraded. As the wavelength of the illumination light L becomes shorter, a finer structure of the observation target can be imaged, whereas scattering increases and the image quality may be degraded. Therefore, in some cases, increasing the wavelength of the illumination light L can reduce scattered light and suppress the image quality degradation. Accordingly, the image acquisition condition determination unit 61B determines a wavelength to be used for the illumination light L in accordance with the shape of the flow channel 29 and characteristics of the flow channel member 74, such as transmittance.

As illustrated in Fig. 9, examples of the image processing condition include conditions regarding the identification of a region of a cell 13 to be subjected to image reconstruction processing in the interference fringe image 31 and the setting of parameters for the image reconstruction processing. As an example of the interference fringe image 31, three interference fringe images 31 of wavelengths λ1, λ2, and λ3 are used.

If a region AR of the cell 13 can be identified in the interference fringe image 31, information that becomes noise, such as information on the flow channel 29 other than the cell 13, can be removed. Accordingly, the image quality of the cell image 32 is improved. The region AR is identified by using, for example, a difference between the refractive index of the flow channel member 74 and the refractive index of the cell 13. Additionally, a difference between the refractive index of the cell 13 and a refractive index of the culture medium 14 may also be used. Based on these differences in refractive index, a region of the interference fringes 67 corresponding to the cell 13 can be identified in the interference fringe image 31.

As an example of the image reconstruction processing for digital holography, a Fourier iterative phase retrieval method or the like is used. The Fourier iterative phase retrieval method requires a large amount of computation and a significant amount of time because it involves iterative calculations that repeat Fourier transforms and inverse Fourier transforms while changing parameters. If the region AR where the cell 13 is present is identified in the interference fringe image 31, the region where the iterative calculations are to be performed can be limited to the region AR, thereby reducing the influence of noise. Accordingly, the image quality of the cell image 32 is also improved. Furthermore, limiting image processing to the region AR also reduces the processing time of the image processing.

As described above, the image acquisition condition determination unit 61B determines an image acquisition condition including an imaging condition and an image processing condition, based on the flow channel information and the observation target information. In the present example, the observation target information is used in addition to the flow channel information, but only the flow channel information may be used. This is because the identification of the region AR and the like can be appropriately performed in some cases by, for example, using an average value or the like of the refractive indices of the cell 13 and the culture medium 14, even if the type of the cell 13 and the type of the culture medium 14 are not specified.

As an example, such flow channel information and observation target information are input to the control device 26 by a manual operation of an operator through the input device 55. The flow channel information may be the values themselves of the dimensions, the transmittance, the refractive index, and the like of the flow channel 29, or may be representative values capable of identifying these values. Examples of the representative values include the model number of the flow channel member 74. Representative values of the transmittance and the refractive index of the flow channel 29 may be the material or the like of the flow channel member 74. In a case where these representative values are input, the control device 26 acquires information corresponding to the representative values, such as the dimensions and the refractive index, from table data or the like. The control device 26 acquires the flow channel information in this way. Similarly, the observation target information is also input to the control device 26 by a manual operation.

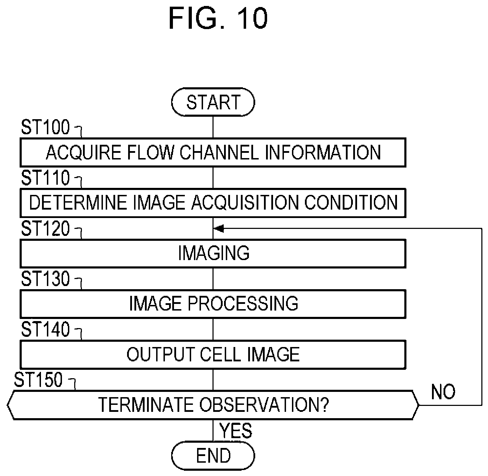

Operations of the above configuration will be described with reference to a flowchart illustrated in Fig. 10. As illustrated in Fig. 10, first, in step ST100, the processor 61 of the control device 26 acquires flow channel information. As the flow channel information, flow channel information relating to the flow channel 29 of the cell culture system 2 in which the cell observation apparatus 11 is used is set. Observation target information is also set in the cell observation apparatus 11 in a similar way. Then, in step ST110, the processor 61 determines an image acquisition condition including an imaging condition and an image processing condition, based on the acquired flow channel information and observation target information.

In the cell culture system 2, after culturing is started, the cell suspension 15 including the cells 13 is extracted from the culture vessel 16 during culturing. The extracted cell suspension 15 flows through the flow channel 29 and is imaged by the imaging unit 27 in step ST120. The processor 61 acquires an interference fringe image 31 of an imaged cell 13. In step ST130, the processor 61 performs image reconstruction processing based on the acquired interference fringe image 31 to reconstruct a cell image 32. In step ST140, the processor 61 outputs the cell image 32 to the display 54. The process proceeds to step ST150, in which the processor 61 determines whether the observation is to be terminated, and repeatedly performs the above processing until the observation ends.

The operator checks the cell 13 appearing in the displayed cell image 32 and ascertains the cell quality. Then, the operator determines a process condition of the culturing process in accordance with the cell quality, and sets the determined process condition in the culture control device 19. Accordingly, the process condition is set and changed according to the cell quality. As a result, appropriate cell culture is performed. Furthermore, the frequency of monitoring for checking the cell quality may be changed in accordance with the cell quality. For example, the number of checks of the cell quality may be increased in a case where the cell quality has not reached a target.

According to the cell observation apparatus 11 according to the technology of the present disclosure, the processor 61 acquires flow channel information, which is information relating to the flow channel 29 through which the cell suspension 15 including the cells 13 flows, and determines, based on the flow channel information, an image acquisition condition for acquiring an image of a cell 13 in the flow channel 29. The image according to the technology of the present disclosure is, for example, the interference fringe image 31 or the cell image 32 representing the three-dimensional shape of the cell 13 in a case where the image is acquired by digital holography technology. Therefore, the image quality of the cell image 32 representing the cell 13 in the flow channel 29 can be improved, compared with a case where the flow channel information is not used for determining an image acquisition condition.

Since the improvement of the image quality of the cell image 32 enables accurate ascertainment of the cell quality, a process condition of the culturing process can be appropriately determined. Observation of the cells 13 flowing through the flow channel 29 is also necessary in a case where the cell quality of cells 13 being cultured is to be ascertained. The technology of the present disclosure is effective for observing the cells 13 in the flow channel 29 because the technology of the present disclosure determines an image acquisition condition using the flow channel information.

In the above embodiment, the image acquisition condition includes at least one of an imaging condition for capturing an image of a cell 13 in the flow channel 29 or an image processing condition for performing image processing. Either the imaging condition or the image processing condition is included, thereby enabling a favorable cell image 32 to be acquired.

In the above embodiment, furthermore, the image processing condition includes identifying the region AR of the cell 13. Accordingly, noise other than the cell 13 can be reduced, thereby providing the cell image 32 having favorable image quality. In a case where an image is acquired by digital holography technology, the range of image processing can be narrowed by identifying the region AR of the cell 13 in the interference fringe image 31. Therefore, the amount of computation and the processing time of the image processing can be reduced.

In the above embodiment, furthermore, the imaging condition includes at least one of the wavelength of the illumination light L for illuminating the flow channel 29 or an exposure condition. Appropriately selecting the wavelength of the illumination light L suppresses scattering, thereby enabling the cell image 32 having favorable image quality to be obtained. The exposure condition is included, thereby enabling the cell image 32 having high contrast and favorable image quality to be acquired.

In the above embodiment, furthermore, the flow channel information includes at least one of information relating to the structure of the flow channel 29 or information relating to characteristics of the flow channel 29. In the above embodiment, an imaging condition or an image processing condition can be appropriately determined, compared with a case where the information relating to the structure or characteristics of the flow channel 29 is absent. For example, in the interference fringe image 31 and the cell image 32, information other than the cell 13, such as the flow channel 29, becomes noise. If the dimensions and shape of the flow channel 29 are determined as the information relating to the structure of the flow channel 29 during image processing, a region of the flow channel 29 in the interference fringe image 31 or the cell image 32 can be easily identified, thereby enabling information on the flow channel 29 as noise to be removed from the interference fringe image 31 or the cell image 32. Further, for example, if the transmittance of the flow channel member 74 is determined as a characteristic of the flow channel 29, the amount of transmission of the illumination light L into the flow channel 29 can be estimated, thereby enabling appropriate exposure control.

In the above embodiment, furthermore, the flow channel information includes, as a characteristic of the flow channel 29, the refractive index of the flow channel member 74 constituting the flow channel 29. If the refractive index of the flow channel member 74 is determined as a characteristic of the flow channel 29, information on the flow channel 29 as noise can be removed from the interference fringe image 31 or the cell image 32, based on the difference in refractive index from the cell 13.

In the above embodiment, furthermore, the cell observation apparatus 11 includes an imaging device (as an example, the imaging unit 27) that captures an image of a cell 13, and the imaging device is an image measurement device using digital holography technology. As described above, in a case where an image measurement device using digital holography technology is used, an image acquisition condition is important for improving image quality and improving image processing efficiency, and thus the technology of the present disclosure is very effective.

In the above embodiment, furthermore, the processor 61 determines the image acquisition condition based on, in addition to the flow channel information, information on at least one of the type of the cell 13 or the type of the culture medium 14 used for the cell suspension 15. In addition to the flow channel information, characteristics (such as transmittance and refractive index) according to a cell type and a culture medium type can be taken into consideration, thereby allowing a more appropriate image acquisition condition to be determined.

The imaging unit 27 is disposed in the observation path 22. The flow channel 29 of the observation path 22 is a flow channel through which the cell suspension 15 extracted from the culture vessel 16 for culturing the cells 13 and including the cells 13 being cultured flows. Therefore, the state of the cells being cultured can be ascertained, and a process condition of the culturing process can be controlled during cell culture.

Since the flow channel 29 of the observation path 22 is a flow channel for observing the cells 13 being cultured, the range of changes in the cell quality of the cells 13 is also broad, unlike the flow channel of the culture product recovery path 21 through which the cultured cells 13 flow. Therefore, in a case where the cells 13 being cultured are observed, the necessity to accurately ascertain cell quality is higher than in a case where the cultured cells 13 are observed, and the technology of the present disclosure is particularly effective.

### Second Embodiment

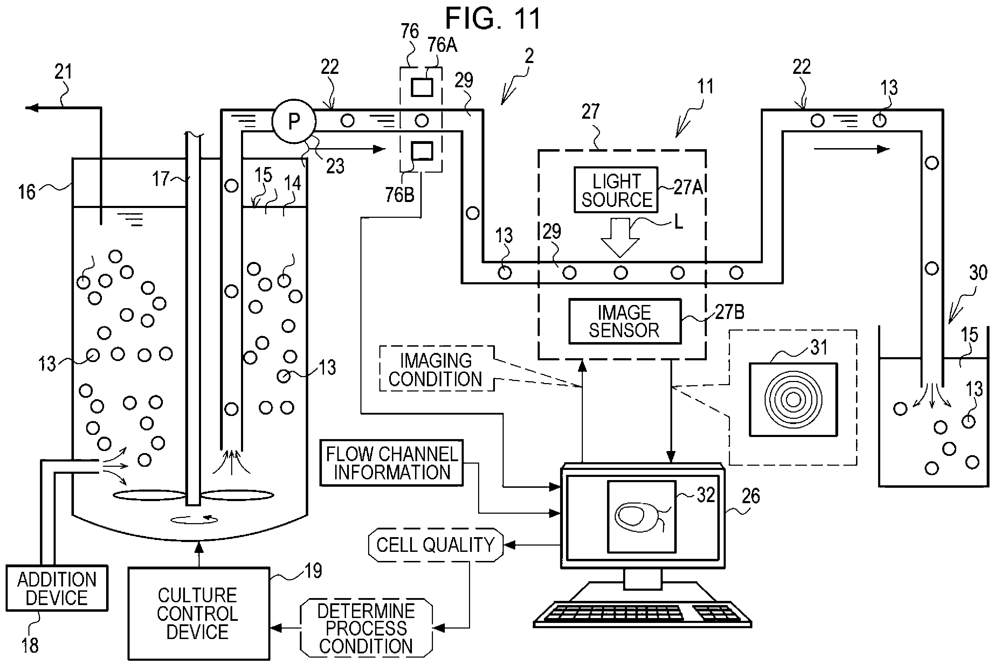

Unlike the first embodiment, a cell observation apparatus 11 according to a second embodiment illustrated in Fig. 11 includes a photosensor 76 disposed on the upstream side of the imaging unit 27 in the observation path 22. Other configurations are similar to those of the first embodiment except that processing using the photosensor 76 is different.

As an example, the photosensor 76 is a transmission-type photosensor in which a light-emitting element 76A and a light-receiving element 76B are disposed facing each other with the flow channel 29 of the observation path 22 interposed therebetween. In the photosensor 76, as is well known, the light-receiving element 76B receives detection light emitted from the light-emitting element 76A. Upon an object such as a cell 13 passing between the light-emitting element 76A and the light-receiving element 76B, part of the detection light is blocked by the object, thereby changing the amount of light received by the light-receiving element 76B. The light-receiving element 76B outputs a detection signal corresponding to the amount of received light to the processor 61. Instead of the transmission-type photosensor, a reflection-type photosensor may be used as the photosensor 76.

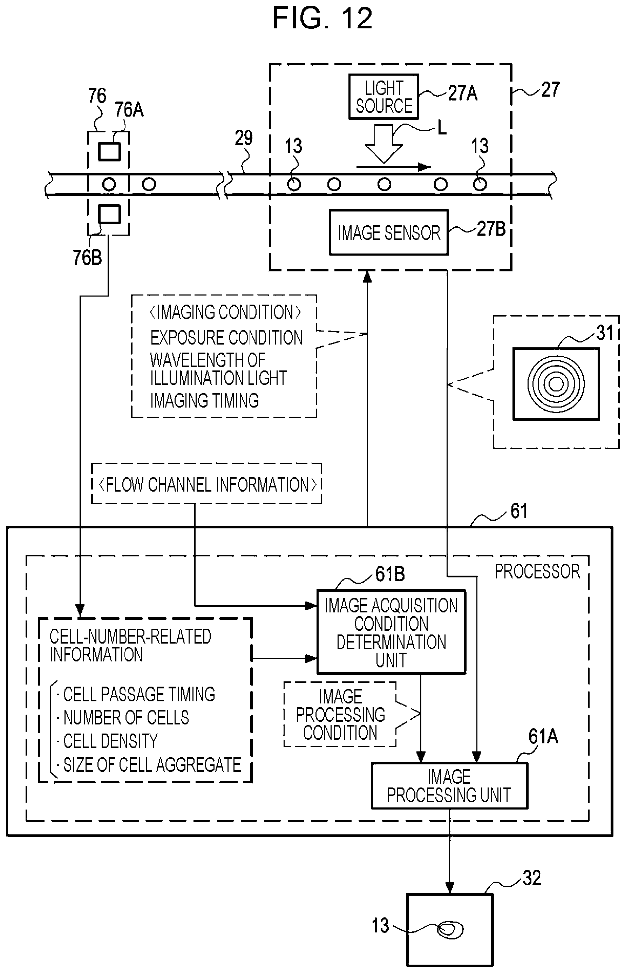

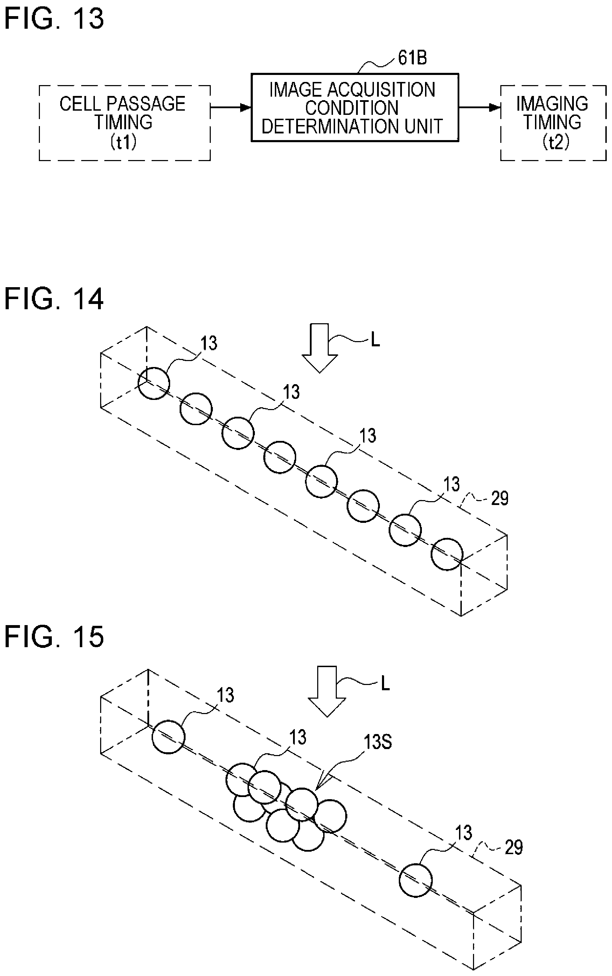

As illustrated in Fig. 12, the processor 61 detects cell-number-related information based on the detection signal received from the photosensor 76. The cell-number-related information includes the passage timing of the cell 13, the number of cells 13 passing within a certain period of time, the density of the cells 13 passing within the certain period of time, the size of a cell aggregate 13S (see Fig. 15) formed by an aggregation of a plurality of cells 13, and the like.

The processor 61 can detect the passage timing of the cell 13 passing through the photosensor 76, based on a change in the detection signal received from the photosensor 76 and corresponding to the amount of received light. Furthermore, by counting changes in the detection signal, the processor 61 can detect the number of cells 13 passing through the photosensor 76. Furthermore, by measuring an average period of the detection signal that periodically changes in a case where a plurality of cells 13 pass through the photosensor 76, the processor 61 can detect a density of a plurality of cells 13 passing within a certain period of time. Furthermore, by measuring a degree to which the detection signal drops and a time during which the detection signal remains dropped, the processor 61 can detect a size of the cell aggregate 13S. This is because a decrease in the amount of light received by the light-receiving element 76B per unit time is larger and the duration of the decrease is longer in a case where the cell aggregate 13S passes than in a case where a single cell 13 passes.

As described above, in the flow channel 29, the photosensor 76 and the processor 61 detect at least one of the passage timing of a cell 13, the number of cells 13, the density of the cells 13, or the size of a cell aggregate in a section on the upstream side of the imaging unit 27 (an example of an imaging device). That is, the photosensor 76 and the processor 61 are an example of a "detection mechanism" according to the technology of the present disclosure. In the processor 61, the image acquisition condition determination unit 61B determines an image acquisition condition based on a detection result of the detection mechanism.

For example, as illustrated in Fig. 13, the image acquisition condition determination unit 61B can determine an imaging timing, which is an imaging condition of the imaging unit 27. Specifically, by detecting a passage timing t1 of a cell 13 passing through the photosensor 76, the image acquisition condition determination unit 61B can determine an imaging timing t2 of the imaging unit 27. The flow velocity of the cell 13 can be estimated from the driving force of the pump 23. Therefore, detecting the passage timing t1 allows the imaging timing t2 to be determined through calculation of the time taken for the cell 13 that has passed through the photosensor 76 to reach the imaging unit 27 located on the downstream side in the flow channel 29. If the imaging timing t2 is determined, imaging can be performed only during a period in which the cell 13 passes through the imaging unit 27. Consequently, unnecessary imaging without any cell 13 can be reduced.

For example, the image acquisition condition determination unit 61B determines an exposure condition as an imaging condition in accordance with the density of the cells 13 or the size of the cell aggregate 13S. As illustrated by way of example in Fig. 14, in a case where a plurality of cells 13 pass at shorter intervals with a higher density than in Fig. 5, the amount by which the illumination light L from the imaging unit 27 is blocked by the cells 13 increases, which may result in a decrease in contrast. As illustrated by way of example in Fig. 15, in a case where the flow channel 29 has a large cross-sectional area, a plurality of cells 13 may aggregate in the flow channel 29 and flow as a single cell aggregate 13S. Also in a case where such a cell aggregate 13S is imaged, the amount by which the illumination light L is blocked is larger than in a case where a single cell 13 is imaged, which may result in a decrease in contrast.

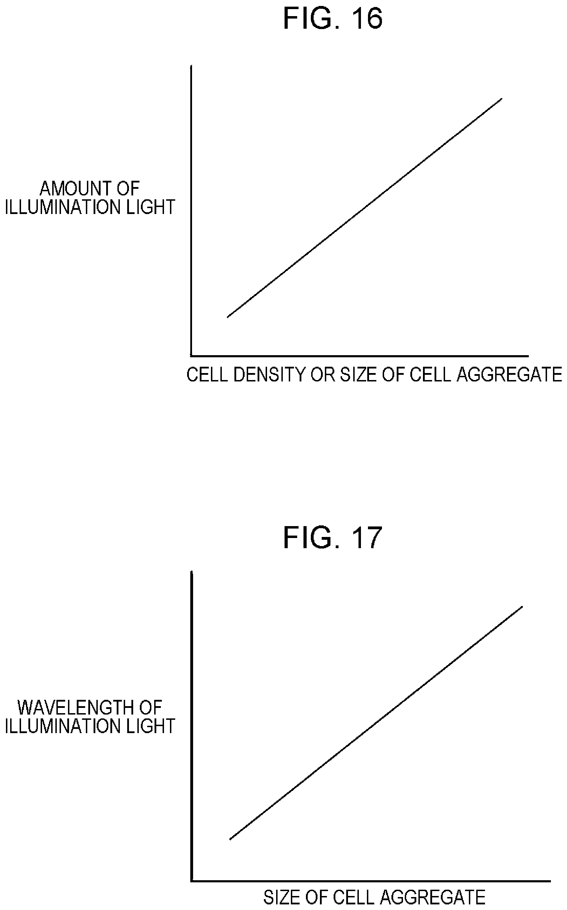

Therefore, as illustrated by way of example in Fig. 16, the image acquisition condition determination unit 61B performs control to increase the amount of the illumination light L in accordance with the density of the cells 13 or the size of the cell aggregate 13S. It is to be understood that the exposure time may be increased instead of increasing the amount of the illumination light L. As a result, an appropriate exposure amount can be obtained regardless of the density of the cells 13 or the like. Consequently, the decrease in contrast is suppressed, resulting in an improvement in the image quality of the cell image 32. For example, the storage 50 stores table data or functions indicating the relationships illustrated in Figs. 16 and 17. While referring to such data, the image acquisition condition determination unit 61B executes control of an exposure condition based on the flow channel information.

As the size of the cell aggregate 13S increases, scattering of the illumination light L increases, and the increase in scattered light may degrade the image quality of the cell image 32. Scattered light may be suppressed by increasing the wavelength of the illumination light L. Therefore, as illustrated by way of example in Fig. 17, the image acquisition condition determination unit 61B increases the wavelength of the illumination light L as the size of the cell aggregate 13S increases.

As described above, by using the photosensor 76, the cell observation apparatus 11 according to the second embodiment can detect, in real time, changes in the state of the cells 13 in the flow channel 29, such as the number and density of cells 13 flowing through the flow channel 29, and can determine an appropriate exposure condition in accordance with the changes in the state of the cells 13.

The processor 61 may perform control to reduce the flow velocity of the cells 13 in accordance with the imaging timing. This can suppress blurring of the cell image 32. The flow velocity of the cells 13 can be controlled by controlling the pump 23.

### Modifications

In the above embodiments, an operator ascertains the cell quality based on the cell image 32 output from the cell observation apparatus 11, and determines a process condition of the culturing process based on the ascertained cell quality. Such a function may be added to the cell observation apparatus 11.

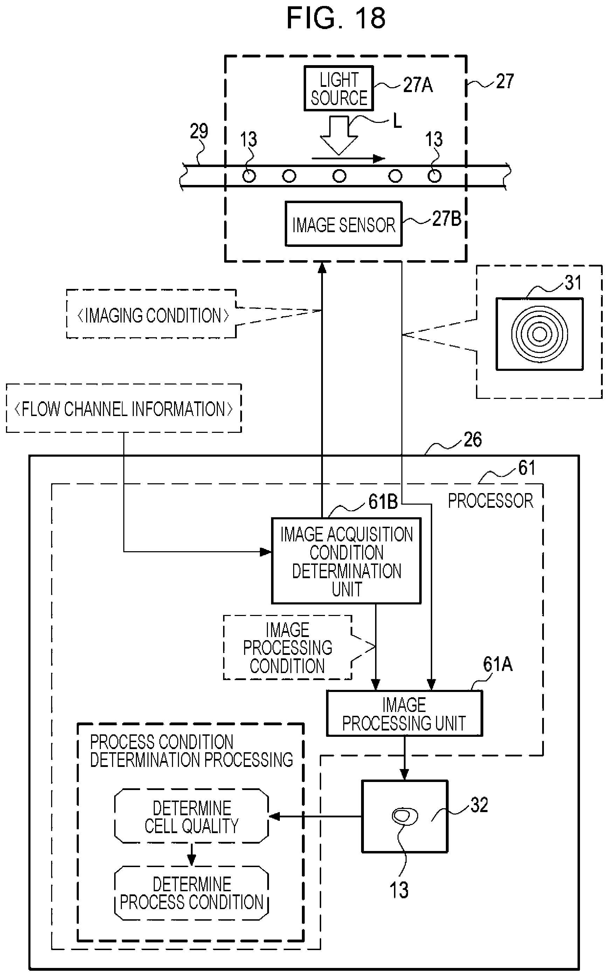

In the cell observation apparatus 11 illustrated in Fig. 18, the processor 61 has a function of determining a process condition of the culturing process for the cells 13 based on the cell image 32. The processor 61 executes process condition determination processing. In the process condition determination processing, the processor 61 performs image analysis on the cell image 32 to determine the cell quality of the cells 13. Based on the determination result, the processor 61 determines a process condition including the above-described culture condition for the cells 13.

While an image measurement device using digital holography technology has been described as an example of the cell observation apparatus 11, a phase-contrast microscope, a brightfield microscope, or the like may also be used as the cell observation apparatus 11. For example, exposure condition control based on the flow channel information is also effective for a phase-contrast microscope and a brightfield microscope.

Various modifications may be made to the hardware configuration of a computer constituting the cell observation apparatus 11. For example, for improvements in processing capability and reliability, the cell observation apparatus 11 may also be constituted by a plurality of computers that are separate in terms of hardware.

As described above, the hardware configuration of the computer of the cell observation apparatus 11 may be changed as appropriate in accordance with performance requirements such as processing capability, safety, and reliability. Furthermore, it is to be understood that, in addition to the hardware, application programs such as the operation program 58 may also be duplicated or stored in a plurality of storage devices in a distributed manner for ensuring safety and reliability.

The above description further discloses the following technologies.

### Appendix 1

A cell observation apparatus including a processor configured to:
acquire flow channel information, the flow channel information being information relating to a flow channel through which a cell suspension including cells flows; and
determine an image acquisition condition for acquiring an image of the cells in the flow channel, based on the flow channel information.

### Appendix 2

The cell observation apparatus according to Appendix 1, wherein the image acquisition condition includes at least one of an imaging condition for capturing the image of the cells in the flow channel or an image processing condition for performing image processing.

### Appendix 3

The cell observation apparatus according to Appendix 2, wherein
the imaging condition includes at least one of a wavelength of illumination light for illuminating the flow channel or an exposure condition.

### Appendix 4

The cell observation apparatus according to any one of Appendices 1 to 3, wherein
the flow channel information includes at least one of information relating to a structure of the flow channel or information relating to a characteristic of the flow channel.

### Appendix 5

The cell observation apparatus according to Appendix 4, wherein
the characteristic of the flow channel includes a refractive index of a flow channel member constituting the flow channel.

### Appendix 6

The cell observation apparatus according to any one of Appendices 1 to 5, including
an imaging device configured to capture the image of the cells, wherein
the imaging device is an image measurement device using digital holography technology.

### Appendix 7

The cell observation apparatus according to any one of Appendices 1 to 6, including:
an imaging device configured to capture the image of the cells; and
a detection mechanism configured to detect passage of the cells in a section on an upstream side of the imaging device in the flow channel and detect at least one of a passage timing of the cells that pass, a number of cells that pass, a density of the cells that pass, or a size of a cell aggregate that passes, wherein
the processor is configured to determine the image acquisition condition based on a detection result of the detection mechanism.

### Appendix 8

The cell observation apparatus according to any one of Appendices 1 to 7, wherein
the processor is configured to determine the image acquisition condition based on, in addition to the flow channel information, information on at least one of a type of the cells or a type of a culture medium used for the cell suspension.

### Appendix 9

The cell observation apparatus according to any one of Appendices 1 to 8, wherein
the processor further has a function of determining a process condition of a culturing process for the cells, based on the image.

### Appendix 10

The cell observation apparatus according to any one of Appendices 1 to 9, wherein
the flow channel is a flow channel through which the cell suspension that is extracted from a culture vessel for culturing the cells and that includes a cell being cultured flows.

### Appendix 11

An operation method for a cell observation apparatus including a processor, the operation method including,
by the processor:
acquiring flow channel information, the flow channel information being information relating to a flow channel through which a cell suspension including cells flows; and
determining an image acquisition condition for acquiring an image of the cells in the flow channel, based on the flow channel information.

### Appendix 12

An operation program for a cell observation apparatus including a processor, the operation program causing the processor to execute a process including:
acquiring flow channel information, the flow channel information being information relating to a flow channel through which a cell suspension including cells flows; and
determining an image acquisition condition for acquiring an image of the cells in the flow channel, based on the flow channel information.

In the above embodiments, the hardware structures of processing units that execute various processes, such as the image processing unit 61A and the image acquisition condition determination unit 61B, may be implemented using various processors described below. As described above, the various processors include the CPU 52, which is a general-purpose processor configured to execute software (the operation program 58) to function as various processing units, and further include a programmable logic device (PLD) that is a processor whose circuit configuration can be changed after manufacture, such as a field programmable gate array (FPGA), a dedicated electric circuit that is a processor having a circuit configuration designed exclusively for executing specific processing, such as an application specific integrated circuit (ASIC), and so on.

A single processing unit may be constituted by one of the various processors, or may be constituted by a combination of two or more processors of the same type or different types (for example, a combination of a plurality of FPGAs, and/or a combination of a CPU and an FPGA). Alternatively, a plurality of processing units may be configured as a single processor.

Examples of configuring a plurality of processing units with a single processor include, first, a form in which, as typified by computers such as a client and a server, a single processor is constituted by a combination of one or more CPUs and software, and the processor functions as the plurality of processing units. The examples include, second, a form in which, as typified by a system on chip (SoC) or the like, a processor is used in which the functions of the entire system including the plurality of processing units are implemented as one integrated circuit (IC) chip. As described above, the various processing units are configured by using one or more of the various processors described above as a hardware structure.

More specifically, the hardware structure of these various processors may be an electric circuit (circuitry) in which circuit elements such as semiconductor elements are combined.

In the technology of the present disclosure, the various embodiments and various modifications described above may be combined as appropriate. It is to be understood that the technology of the present disclosure is not limited to the above embodiments, and that various configurations may be adopted without departing from the gist thereof. Furthermore, the technology of the present disclosure encompasses not only a program but also a storage medium storing the program in a non-transitory manner. Examples of the storage medium include a non-transitory computer-readable storage medium such as a universal serial bus (USB) memory, a flexible disk, and a compact disc read only memory (CD-ROM). The program may be provided online via a network such as the Internet. The technology of the present disclosure encompasses not only the program but also a program product. The program product includes any form of product for providing the program. Similarly to the program, the program product may be provided by being stored in a non-transitory computer-readable storage medium, or may be provided online.

The description and drawings presented above provide detailed descriptions of portions according to the technology of the present disclosure and are merely examples of the technology of the present disclosure. For example, the descriptions related to the configurations, functions, operations, and effects described above are descriptions related to an example of the configurations, functions, operations, and effects of portions according to the technology of the present disclosure. Thus, it goes without saying that unnecessary portions may be deleted or new elements may be added or substituted in the description and drawings presented above without departing from the gist of the technology of the present disclosure. To avoid complexity and facilitate understanding of portions according to the technology of the present disclosure, descriptions related to common general technical knowledge and the like, for which no specific explanation is required to implement the technology of the present disclosure, are omitted in the description and drawings presented above.

In this specification, "A and/or B" is synonymous with "at least one of A or B". That is, "A and/or B" means only A, only B, or a combination of A and B. In this specification, furthermore, a concept similar to that of "A and/or B" is applied also to the expression of three or more matters in combination with "and/or".

The disclosure of JP2024-026481 filed on February 26, 2024 is incorporated herein by reference in its entirety. All publications, patent applications, and technical standards mentioned herein are incorporated herein by reference to the same extent as if each individual publication, patent application, and technical standard were specifically and individually indicated to be incorporated by reference.

## Claims

1. A cell observation apparatus comprising a processor configured to:
acquire flow channel information, the flow channel information being information relating to a flow channel through which a cell suspension including cells flows; and
determine an image acquisition condition for acquiring an image of the cells in the flow channel, based on the flow channel information.

2. The cell observation apparatus according to claim 1, wherein the image acquisition condition includes at least one of an imaging condition for capturing the image of the cells in the flow channel or an image processing condition for performing image processing.

3. The cell observation apparatus according to claim 2, wherein
the imaging condition includes at least one of a wavelength of illumination light for illuminating the flow channel or an exposure condition.

4. The cell observation apparatus according to claim 1, wherein
the flow channel information includes at least one of information relating to a structure of the flow channel or information relating to a characteristic of the flow channel.

5. The cell observation apparatus according to claim 4, wherein
the characteristic of the flow channel includes a refractive index of a flow channel member constituting the flow channel.

6. The cell observation apparatus according to claim 1, further comprising
an imaging device configured to capture the image of the cells, wherein
the imaging device is an image measurement device using digital holography technology.

7. The cell observation apparatus according to claim 1, further comprising:
an imaging device configured to capture the image of the cells; and
a detection mechanism configured to detect passage of the cells in a section on an upstream side of the imaging device in the flow channel and detect at least one of a passage timing of the cells that pass, a number of cells that pass, a density of the cells that pass, or a size of a cell aggregate that passes, wherein
the processor is configured to determine the image acquisition condition based on a detection result of the detection mechanism.

8. The cell observation apparatus according to claim 1, wherein
the processor is configured to determine the image acquisition condition based on, in addition to the flow channel information, information on at least one of a type of the cells or a type of a culture medium used for the cell suspension.

9. The cell observation apparatus according to claim 1, wherein
the processor further has a function of determining a process condition of a culturing process for the cells, based on the image.

10. The cell observation apparatus according to claim 1, wherein
the flow channel is a flow channel through which the cell suspension that is extracted from a culture vessel for culturing the cells and that includes a cell being cultured flows.

11. An operation method for a cell observation apparatus including a processor, the operation method comprising,
by the processor:
acquiring flow channel information, the flow channel information being information relating to a flow channel through which a cell suspension including cells flows; and
determining an image acquisition condition for acquiring an image of the cells in the flow channel, based on the flow channel information.

12. An operation program for a cell observation apparatus including a processor, the operation program causing the processor to execute a process comprising:
acquiring flow channel information, the flow channel information being information relating to a flow channel through which a cell suspension including cells flows; and
determining an image acquisition condition for acquiring an image of the cells in the flow channel, based on the flow channel information.
